# EUROPEAN PATENT SPECIFICATION

(11) **EP 3 554 471 B1**
(45) Date of publication and mention of the grant of the patent: **30.04.2025**
(21) Application number: 16924473.8
(22) Date of filing: 19.12.2016
(51) Int. Cl.: A61K 8/9794, A61K 8/02, A61K 8/73, A61Q 11/00

(54) **ORAL CARE COMPOSITIONS INCLUDING CHARCOAL**
MUNDPFLEGEZUSAMMENSETZUNGEN MIT HOLZKOHLE
COMPOSITIONS DE SOIN BUCCAL COMPRENANT DU CHARBON DE BOIS

(43) Date of publication of application: 23.10.2019
(73) Proprietor: Colgate-Palmolive Company, New York, NY 10022 (US)
(72) Inventor: ZHENG, Zhuoli, Guangzhou Guangdong 510730 (CN); XU, Yun, Langhorne Pennsylvania 19047 (US)
(74) Representative: Wibbelmann, Jobst
(86) International application number: PCT/CN2016/110743
(87) International publication number: WO 2018/112697

(56) References cited:
- EP-A2- 1 317 916
- CN-A- 103 417 390
- KR-A- 20050 025 218
- US-A1- 2016 243 017
- US-B1- 6 669 929
- DATABASE GNPD [online] MINTEL; 3 March 2016 (2016-03-03), ANONYMOUS: "Bamboo Charcoal and Mint Whitening Toothpaste", XP055707952, retrieved from www.gnpd.com Database accession no. 3841713

## Description

### BACKGROUND

Conventional oral care products or compositions (*e.g.*, toothpastes, whitening gels, *etc.*) often include or incorporate functional, aesthetic, and/or ornamental elements or features to enhance consumer acceptance and/or experience. For example, conventional dentifrices often incorporate colors or colored elements to provide an aesthetic effect thatconsumers find pleasing, which promotes the use of the dentifrices. Conventional oral care products may also incorporate functional, aesthetic, and/or ornamental elements to distinguish or identify products in the marketplace having particular properties. For example, substantially clear dentifrices, such as toothpastes and gels, often incorporate colored elements to indicate the presence of flavors.

While the incorporation of the functional, aesthetic, and/or ornamental elements into conventional oral care products have proven to enhance consumer acceptance and distinguish products in the marketplace, the type, selection, or number of the elements that have been successfully incorporated into the oral care products is limited. For example, activated carbon or charcoal is recognized by consumers for its ability to naturally adsorb or bind unwanted impurities, toxins, and/or chemicals. The incorporation of charcoal into oral care products, such as substantially clear toothpastes, however, eliminates the transparency or clarity of the toothpastes and results in black or dark toothpastes that are neither visually appealing or accepted by consumers.

What is needed, then, are improved oral care products incorporating activated carbon or charcoal for improved aesthetics and/or therapeutic effects. KR 2005 0025218 A discloses a preparation method of a complex granule additive containing active carbon for toothpaste, which complex granule additive has sufficient strength not to be destroyed during the toothpaste production, prevents color spreading of active carbon therein, and effectively removes bad breath, plaque and tartar. US 2016/243017 A1 discloses a composition for delivery of a payload to an oral cavity, wherein the composition incorporates a payload and is stable under slightly aqueous conditions, but dissolves when exposed to moderate or highly aqueous conditions, and/or mechanical action, releasing the payload, wherein the composition can be used alone or as a component in a toothpaste formulation. US 6 669 929 B1 discloses a dentifrice composition comprising an orally acceptable vehicle having distributed therein water hydratable film flakes having a matrix comprised of a water soluble hydroxy alkyl cellulose polymer and a starch, and having entrained therein a constituent selected from therapeutic, cosmetic and decorative materials. EP 1 317 916 A2 discloses a film, particularly an oral film, which contains starch as the main component, wherein such film is useful for delivering a variety of agents to humans and other animals to produce a therapeutic, organoleptic, pharmacological, agricultural or cosmetic effect, including breath fresheners, flavors, fragrances and pharmaceuticals. The database entry on "Bamboo Charcoal and Mint Whitening Toothpaste", XP055707952, retrievable from www.gnpd.com database, discloses a bamboo charcoal and mint whitening toothpaste.

### BRIEF SUMMARY

This summary is intended merely to introduce a simplified summary of some aspects of one or more embodiments of the present disclosure. Further areas of applicability of the present invention will become apparent from the detailed description provided hereinafter. This summary is not an extensive overview, nor is it intended to identify key or critical elements of the present teachings, nor to delineate the scope of the disclosure. Rather, its purpose is merely to present one or more concepts in simplified form as a prelude to the detailed description below.

The foregoing and/or other aspects and utilities embodied in the present disclosure may be achieved by providing an oral care composition, including a dentifrice; and a film suspended in the dentifrice, wherein the film includes charcoal as further defined in the claims.

The film further includes carboxymethyl cellulose and starch as defined in the claims, optionally methacrylates, polyvinylpyrollidone, and mixtures thereof.

The film includes carboxymethyl cellulose as defined in the claims.

In another embodiment, the charcoal is present in an amount of from about 1 wt% to about 10 wt%, based on a total weight of the film.

In another embodiment, the charcoal is present in an amount of from about 2 wt% to about 6 wt%, based on a total weight of the film.

In another embodiment, the charcoal is derived from a natural source.

In another embodiment, the charcoal has a particle size less than or equal to 75 µm.

In another embodiment, the charcoal has a particle size of from about 3 µm to about 9 µm.

The film further comprises starch as defined in the claims.

In another embodiment, the starch is corn starch.

In another embodiment, the film does not include titanium dioxide.

In another embodiment, the film does not include any dyes.

In another embodiment, the film does not include any pigments.

Further areas of applicability of the present invention will become apparent from the detailed description provided hereinafter. It should be understood that the detailed description and specific examples, while indicating some preferred aspects of the invention, are intended for purposes of illustration only and are not intended to limit the scope of the invention as defined in the claims.

### DETAILED DESCRIPTION

The following description of various preferred aspect(s) is merely exemplary in nature and is in no way intended to limit the invention as defined in the claims, its application, or uses.

As used throughout, ranges are used as shorthand for describing each and every value that is within the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

Additionally, all numerical values are "about" or "approximately" the indicated value, and take into account experimental error and variations that would be expected by a person having ordinary skill in the art. It should be appreciated that all numerical values and ranges disclosed herein are approximate values and ranges, whether "about" is used in conjunction therewith.

The present inventors have surprisingly and unexpectedly discovered that a film formed from a matrix including a water soluble polymer, such as a methylcellulose, and starch sufficiently suspends and/or incorporates charcoal or activated carbon into the matrix thereof. Particularly, a matrix including a hydroxyalkyl methylcellulose polymer and starch is capable of sufficiently entraining, suspending, or otherwise incorporating charcoal or activated carbon therein. The film formed from the matrix and including the charcoal is suspended in the dentifrice as defined in the claims, generally, films can be mixed, or otherwise incorporated into a substantially clear dentifrice to produce an oral care composition, where the charcoal does not bleed into the dentifrice until the film is ruptured upon use as defined in the claims.

### Compositions

Compositions disclosed herein comprise a film suspended in the dentifrice composition as further defined in the claims. The film may be a water hydratable film including a homogenous mixture or matrix of a water soluble polymer, such as a hydroxyalkyl cellulose polymer, and starch. The homogenous mixture or matrix of the film may sufficiently entrain, suspend, or otherwise incorporate charcoal or activated carbon. The charcoal is suspended or entrained within the film as defined in the claims. The charcoal may be a therapeutic, cosmetic, and/or a decorative agent. For example, the charcoal dispersed in the film may provide therapeutic benefits, such as detoxification. In another example, the charcoal dispersed in the film does not provide any therapeutic benefits, but instead communicates or informs the consumer of the presence of charcoal in the dentifrice. For example, the charcoal dispersed in the film may be only a decorative agent, thereby providing an aesthetically pleasing and/or consumer acceptable decorative dentifrice that enhances consumer experience and/or acceptance.

The film includes carboxymethyl cellulose and starch as defined in the claims, optionally further components selected from one or more of hydroxyalkyl cellulose polymers, such as hydroxypropyl methyl cellulose (HPMC), hydroxypropyl cellulose, hyrdoxyethyl cellulose, methyl cellulose, carboxymethyl cellulose, and mixtures or combinations thereof. In a preferred embodiment, the water soluble polymer includes one or more hydroxyalkyl cellulose polymers. Illustrative hydroxyalkyl cellulose polymers may be or include, but are not limited to, hydroxypropyl methyl cellulose, hydroxyethylpropyl cellulose, hydroxybutyl methyl cellulose, carboxymethyl cellulose, and the like, and mixtures or combinations thereof. In a preferred embodiment, the hydroxyalkyl cellulose polymer is a low viscosity hydroxylpropyl methyl cellulose polymer (HPMC). For example, the HPMC polymer may have a viscosity of from about 1 millipascal seconds (mPa.s) to about 40 mPa.s, as determined as a 2 wt% aqueous solution of the HPMC at 20°C using a Ubbelohde tube viscometer. In a preferred embodiment, the HPMS has a viscosity of about 3 mPa.s to about 20 mPa.s at 20°C. HPMC is commercially available from Dow Chemical Company as METHOCEL^{™} E5 LV. METHOCEL^{™} E5 LV is a USP grade, low viscosity HPMC having 29.1% methoxyl groups and 9% hydroxyproxyl group substitutions. In a 2 wt% solution, the METHOCEL^{™} has a viscosity of 5.1 mPa.s at 20°C as measured with a Ubbelohde tube viscometer.

The amount or concentration of the hydroxyalkyl cellulose polymers present in the film of the dentifrice composition may vary widely. In at least one embodiment, the amount of the hydroxyalkyl cellulose polymers present in the film of the dentifrice composition may be greater than or equal to about 10 wt% and less than or equal to about 75 wt% based on a dry weight of the film. For example, the amount of the hydroxyalkyl cellulose polymers present in the film of the dentifrice composition may be from about 10 wt%, about 15 wt%, about 20 wt%, about 25 wt%, about 30 wt%, about 35 wt%, or about 40 wt% to about 45 wt%, about 50 wt%, about 55 wt%, about 60 wt%, about 65 wt%, about 70 wt%, or about 75 wt% based on a dry weight of the film. In another example, the amount of the hydroxyalkyl cellulose polymers present in the film of the dentifrice composition may be greater than greater than 10 wt%, greater than 15 wt%, greater than 20 wt%, greater than 25 wt%, greater than 30 wt%, greater than 35 wt%, greater than 40 wt%, greater than 45 wt%, greater than 50 wt%, greater than 55 wt%, greater than 60 wt%, greater than 65 wt%, greater than 70 wt%, or greater than 75 wt% based on a dry weight of the film. In at least one embodiment, the amount of the hydroxyalkyl cellulose polymers present in the film of the dentifrice composition may be greater than 60 wt% based on a dry weight of the film. For example, the amount of the hydroxyalkyl cellulose polymers present in the film of the dentifrice composition may be greater than or equal to 68 wt% based on a dry weight of the film. In a preferred embodiment, the amount of the hydroxyalkyl cellulose polymers present in the film of the dentifrice composition may be about 69 wt% based on a dry weight of the film.

As discussed above, the film of the dentifrice composition comprises charcoal, carboxymethyl cellulose and starch as defined in the claims. The starches may be or include but are not limited to, pregelatenized starches. For example, the starches may be a water swellable, physically modified and pregelatenized starch. The starches may be configured to increase the relative stiffness of the hydroxyalkyl methyl cellulose matrix or homogenous mixture. The starch of the film may be prepared by heating granular starch in the presence of water and optionally an organic solvent at a temperature not greater than 10°C above the gelatinization temperature thereof. The starch obtained from the heating of the granular starch may then be dried to obtain the water swellable, physically modified and pregelatenized starch. In a preferred embodiment, the pregelatinized starch is corn starch. In a more preferred embodiment, the pregelatinized starch is a pregelatinized, stabilized and crosslinked waxy maize starch.

The amount or concentration of the starches present in the film of the dentifrice composition may vary widely. In at least one embodiment, the amount of the starches present in the film of the dentifrice composition may be greater than or equal to about 10 wt% to about 26 wt% based on a dry weight of the film. For example, the amount of the starches present in the film of the dentifrice composition may be from about 10 wt%, about 12 wt%, about 14 wt%, or about 16 wt% to about 18 wt%, about 20 wt%, about 22 wt%, about 24 wt%, or about 26 wt%. In another example, the amount of the starches present in the film of the dentifrice composition may be from about 10 wt% to about 26 wt%, about 12 wt% to about 24 wt%, about 14 wt% to about 22 wt%, or about 16 wt% to about 20 wt%. In a preferred embodiment, the amount of the starches present in the film of the dentifrice composition may be about 16 wt% to about 19 wt%. For example, the amount of the starches present in the film of the dentifrice composition may be about 16 wt%. In another example, the amount of the starches present in the film of the dentifrice composition may be about 18.7 wt%. In at least one embodiment, a ratio of the hydroxyalkyl methyl cellulose to starch may be from about 1:3 to about 4:1.

The charcoal suspended or entrained within the film may have particle sizes less than or equal to 75 µm. For example, the particles sizes of the charcoal may be substantially (e.g., at least 90% or 95%) less than or equal to 75 µm. For example, at least 95% of the particles of charcoal may have a size less than or equal to 75 µm, less than or equal to 65 µm, less than or equal to 60 µm, less than or equal to 55 µm, less than or equal to 50 µm, less than or equal to 45 µm, or less than or equal to 50 µm. In a preferred embodiment, the particle size of the charcoal is less than or equal to 10 µm. For example, the particle size of the charcoal may be from about 3 µm to about 9 µm. In another example, the charcoal may have a mean particle size of about 5.5 or 5.6 µm. In a preferred embodiment, the charcoal is at least 90 wt% carbon, preferably at least 95 wt% carbon, and less than 10 wt% ash, preferably less than 5 wt% ash. The charcoal may be derived from any suitable source. For example, the charcoal may be derived from a natural source or origin. In at least one example, the charcoal is derived from natural bamboo. For example, the charcoal may be derived from burning bamboo without any chemical treatment (e.g., natural). The charcoal dispersed in the film may be pharmacologically and physiologically non-toxic. The charcoal may or may not have a porous structure to provide adsorption properties.

The amount or concentration of the charcoal present in the film of the dentifrice composition may vary widely. In at least one embodiment, the amount of the charcoal present in the film of the dentifrice composition may be greater than or equal to about 1 wt% to about 20 wt% on a dry matter basis or from about 1 wt% to about 10 wt% on a dry matter basis. For example, the amount of the charcoal present in the film of the dentifrice composition may be from about 1 wt%, about 2 wt%, about 3 wt%, about 4 wt%, about 5 wt%, about 6 wt%, about 7 wt%, about 8 wt%, about 9 wt%, or about 10 wt% to about 12 wt%, about 13 wt%, about 14 wt%, about 15 wt%, about 16 wt%, about 17 wt%, about 18 wt%, about 19 wt%, or about 20 wt%. In another example, the amount of the charcoal present in the film of the dentifrice composition may be from about 1 wt% to about 20 wt%, about 2 wt% to about 19 wt%, about 3 wt% to about 18 wt%, about 4 wt% to about 17 wt%, about 5 wt% to about 16 wt%, about 6 wt% to about 15 wt%, about 7 wt% to about 14 wt%, about 8 wt% to about 13 wt%, or about 9 wt% to about 12 wt%. In yet another example, the amount of the charcoal present in the film of the dentifrice composition may be from about 1 wt%, about 2 wt%, about 3 wt%, about 4 wt%, about 5 wt%, or about 6 wt% to about 7 wt%, about 8 wt%, about 9 wt%, about 10 wt%, about 11 wt%, or about 12 wt%. In another example, the amount of the charcoal present in the film of the dentifrice composition may be from about 1 wt% to about 12 wt%, about 2 wt% to about 11 wt%, about 3 wt% to about 10 wt%, about 4 wt% to about 9 wt%, about 5 wt% to about 8 wt%, or about 6 wt% to about 7 wt%.

In at least one embodiment, the film does not include any one or more of titanium dioxide, titanium dioxide coated mica (TIMIRON^{®}), chromium oxide greens, ultramarine blues and pinks and ferric oxides as well as water insoluble dye lakes prepared by extending calcium or aluminum salts of FD&C dyes on alumina such as FD&C Green #1 lake, FD&C Blue #2 lake, FD&C R&D #30 lake and FD&C # Yellow 15 lake. In another embodiment, the film includes titanium dioxide and/or titanium dioxide coated mica (TIMIRON^{®}). In at least one embodiment, the film does not include any dyes and/or pigments.

The film matrix may be prepared by mixing, combining, or otherwise contacting the hydroxyalkyl methyl cellulose polymer, the starch, and a compatible solvent with one another to form a film forming composition. The hydroxyalkyl methyl cellulose polymer and the starch may be dissolved or dispersed in the compatible solvent. Optionally, the starch and/or the hydroxyalkyl methyl cellulose polymer are mixed, dispersed, combined, or otherwise contacted with a compatible solvent, such as water, to form separate starch and/or hydroxyalkyl methyl cellulose polymer fluids or solutions before combining with one another. The hydroxyalkyl methyl cellulose may be mixed or combined with water and heated to aid the dispersion or solvation of the hydroxyalkyl methyl cellulose polymer in the water. An alkylene glycol, such as propylene glycol, and/or a surfactant or emulsifier, such as TWEEN^{®} 80, may be added to the heated hydroxyalkyl methyl cellulose polymer solution prior to combining the hydroxyalkyl methyl cellulose polymer solution with the starch solution.

The method for preparing the film matrix may also include contacting the charcoal with the film forming composition. The hydroxyalkyl methyl cellulose polymer, the starch, the compatible solvent, and the charcoal are combined with one another. The hydroxyalkyl methyl cellulose polymer, the starch, and the charcoal can be combined with one another to form a mixture, such as a powdered mixture, and the powdered mixture is combined with the compatible solvent. The hydroxyalkyl methyl cellulose polymer, the starch, and the compatible solvent can be combined with one another to form the film forming composition, and the charcoal is mixed, dispersed, or otherwise contacted with the film forming composition.

The method for preparing the film matrix may also include casing the film forming composition, including the charcoal dispersed therein, on a releasable carrier or substrate and drying the film forming composition to form a sheet of the film or film matrix material. The substrate may have a surface tension that allows the film forming composition to coat or spread evenly without forming a destructive bond between the film and the substrate. Illustrative substrates may be or include, but are not limited to, glass, stainless steel, TEFLON^{®}, polyethylene-impregnated paper, and the like. In at least one embodiment, the film forming composition may be dried to the film with heat. For example, the film forming composition may be dried in a drying oven, drying terminal, vacuum drier, or any other suitable drying system/equipment that does not adversely affect the components of the film.

A thickness of the film formed may vary widely. In at least one embodiment, the film may have a thickness greater than or equal to about 28 µm and less than or equal to about 60 µm.

The film formed from drying the film forming composition may be subsequently shaped into flakes. For example, the film may be cut and/or punched into one or more shaped flakes having a particle size from about 0.254 mm (about 0.01 inches) to about 12.7 mm (about 0.50 inches), preferably from about 2.032 mm (about 0.08 inches) to about 6.35 mm (0.25 inches).

In at least one embodiment, the film may include a protective barrier overcoat configured to increase a relative stability of the flakes. For example, prior to shaping the flakes via punching or cutting, the film may be coated with a protective barrier overcoat, such as a food grade shellack or an ethyl cellulose, to increase the relative stability of the flakes formed from the film.

The dentifrice or base dentifrice may be substantially clear. As used herein, the expression "substantially clear" may refer to a dentifrice (e.g., toothpaste and/or gel) that is translucent or transparent. In at least one embodiment, the base dentifrice may include an orally acceptable vehicle, including a water-phase with a humectant, which is preferably glycerine or sorbitol or an alkylene glycol, such as polyethylene glycol or propylene glycol, where water is present in amount of about 5 wt% to about 10 wt% and the glycerine, sorbitol, and/or the alkylene glycol ingredients is present in an amount of about 30 wt% to about 80 wt% of the dentifrice, more typically about 50 wt% to about 70 wt% of the dentifrice.

The dentifrice may also include one or more thickeners or gelling agents. The one or more thickeners or gelling agents may be inorganic, organic, natural, and/or synthetic thickeners. The thickeners present are present in the dentifrice composition in an amount or concentration sufficient to form an extrudable, shape-retaining dentifrice that can be extruded from a tube onto a toothbrush without falling between the bristles of the toothbrush. In at least one embodiment, the amount or concentration of the thickeners present is from about 0.10 wt% to about 5 wt%, preferably about 0.2 wt% to about 1 wt%. Illustrative thickeners or gelling agents may include, but are not limited to, inorganic thickening silicas, such as amorphous silicas, which are available from Huber Corporation under the trade designation ZEODENT^{™} 165, Irish moss, iota-carrageenan, gum tragacanth, polyvinylpyrrolidone, and the like, and mixtures or combinations thereof.

In at least one embodiment, the dentifrice or base dentifrice may include one or more polishing agents. Illustrative polishing agents may be or include, but are not limited to, silica, calcined alumina, sodium bicarbonate, calcium carbonate, dicalcium phosphate, calcium pyrophosphate, and the like, and mixture or combinations thereof. In at least one embodiment, at least one of the polishing agents is visually and/or substantially clear. For example, at least one of the polishing agents may be or include a colloidal silica, such as ZEODENT^{™} 115, which is commercially available from Huber Corporation. The polishing agent may also be or include an alkali metal aluminosilicate complex having refractive indices close to the refractive indices of gelling agents and liquids (e.g., water, humectants, etc.) utilized in the dentifrice. The amount of the polishing agents present in the dentifrice or base dentifrice composition may be from about 3 wt% to about 50 wt% based on a total weigh of the dentifrice composition.

In at least one embodiment, the dentifrice or base dentifrice may include one or more surfactants configured to increase prophylactic action and/or render the dentifrice more cosmetically acceptable. The one or more surfactants may be or include, anionic surfactants, cationic surfactants, and the like. Illustrative surfactants may include, but are not limited to, water-soluble salts of higher fatty acid monoglyceride monosulfates, such as the sodium salt of the monsulfated monoglyceride of hydrogenated coconut oil fatty acids, cocamidopropyl betaine, higher alkyl sulfates such as sodium lauryl sulfate, alkyl aryl sulfonates, such as sodium dodecyl benzene sulfonate, higher alkyl sulfoacetates, sodium lauryl sulfoacetate, higher fatty acid esters of 1,2-dihydroxy propane sulfonate, and the substantially saturated higher aliphatic acyl amides of lower aliphatic amino carboxylic acid compounds, such as those having 12 to 16 carbons in the fatty acid, alkyl or acyl radicals, and the like, and mixtures or combinations thereof. Illustrative amides may be or include, but are not limited to, N-lauroyl sarcosine, and the sodium, potassium, and ethanolamine salts of N-lauroyl, N-myristoyl, or N-palmitoyl sarcosine. The amount or concentration of the surfactants present in the dentifrice or base dentifrice composition may be from about 0.3 wt% to about 5 wt%, preferably about 0.5 wt% to about 2.0 wt% based on a total weight of the dentifrice.

A method for preparing the dentifrice may include mixing, combining, or otherwise contacting water, humectants, such as glycerin, sorbitol, and/or polyethylene glycol to form a homogenous gel phase, and contacting a polishing agent with the gel phase. The method may also include adding a thickener, flavors, and/or surfactants to the gel phase to form the dentifrice. The flakes of the film can be added to the gel phase. The flakes can be added to the dentifrice. For example, the flakes are added to the dentifrice last to minimize the amount of shear stress applied to the flakes during prior mixing steps. The amount or concentration of the film (e.g., in the form of flakes) present in the dentifrice or base dentifrice may vary widely. In at least one embodiment, the amount of the film present in the dentifrice may be from about 0.05 wt% to about 1.0 wt%, preferably about 0.1 wt% to about 0.5 wt% by weight of the dentifrice composition.

In at least one embodiment, the dentifrice in which the flakes of the film are suspended is substantially clear. Accordingly, the dentifrice incorporating the flakes appear to include charcoal strips or flakes suspended or entrained within the film, which indicates the presence of charcoal to the consumer, thereby enhancing the consumer experience and/or acceptance. The flakes of the film suspended in the dentifrice may be rupturable. For example, the flakes of the film may be rupturable when brushing teeth with the dentifrice. In another example, the mechanical agitation created during brushing may rupture the flakes, thereby releasing the charcoal into the dentifrice. The release of the charcoal into the dentifrice may indicate the presence of charcoal in the dentifrice to the consumer, thereby increasing consumer acceptance and/or enhancing the consumer experience of the dentifrice. The rupture of the flakes may also release the charcoal onto the surfaces of the oral cavity, such as the teeth and gums, thereby topically applying the charcoal to the surfaces of the oral cavity.

In at least one embodiment, the charcoal may be maintained substantially separate from the dentifrice ingredients or components during manufacture and storage, while subsequently being released when the dentifrice is applied topically to surfaces of the oral cavity. Accordingly, the charcoal contained in the flakes suspended in the dentifrice does not bleed into the dentifrice components until ruptured as defined in the claims, thereby maintaining the dentifrice as a substantially clear toothpaste or gel.

### EXAMPLES

The examples and other embodiments described herein are exemplary and not intended to be limiting in describing the full scope of compositions and methods of this disclosure. Equivalent changes, modifications and variations of specific embodiments, materials, compositions and methods may be made within the scope of the present disclosure, with substantially similar results.

### Example 1

Films 1A-7A were prepared by combining the ingredients/components according to Table 1. To prepare each of the films 1A-7A, starch was combined with water in a mixer for at least 5 minutes. Water was added to a separate mixer and heated to a temperature of up to 75°C. After heating, titanium dioxide, if any, was added to the hot water and mixed for at least 5 minutes. The hydroxyalkyl cellulose polymer(s) was/were then added into the hot water and mixed for at least 20 minutes or until a homogenous solution was obtained. The water was maintained at a temperature of at least 70°C when adding the hydroxyalkyl cellulose polymer(s). The starch solution was then added to the hydroxyalkyl cellulose polymer solution and mixed for an additional 20 minutes. Propylene glycol and TWEEN^{®} 80 were then added and mixed for at least 5 minutes to prepare the film forming composition. The film forming composition was then cast on a polyethylene coated paper at 25°C and dried at 110 °C to form a solid thin film. The final compositions of each of the dried solid thin films are summarized in Table 2.

**Table 1**

| **Film Forming Compositions** | | | | | | | |
|---|---|---|---|---|---|---|---|
| **Ingredient** | **1A** | **2A** | **3A** | **4A** | **5A** | **6A** | **7A** |
| DI water (wt%) | 79.84 | 80.66 | 80.43 | 80.21 | 80.01 | 78.63 | 78.13 |
| Starch (wt%) | 3.84 | 3.84 | 3.84 | 3.84 | 3.84 | 4.13 | 4.13 |
| Titanium Dioxide (wt%) | 0.96 | 0.00 | 0.00 | 0.00 | 0.00 | 0.00 | 0.50 |
| Hydroxyalkyl Cellulose Polymer(s) (wt%) | 12.49 | 12.49 | 12.49 | 12.49 | 12.49 | 13.41 | 13.41 |
| Propylene Glycol (wt%) | 1.92 | 1.92 | 1.92 | 1.92 | 1.92 | 2.06 | 2.06 |
| Surfactant(s) (wt%) | 0.48 | 0.48 | 0.48 | 0.48 | 0.48 | 0.52 | 0.52 |
| CI Pigment Black 7 (wt%) | 0.41 | -- | -- | -- | -- | -- | -- |
| Charcoal Activated Carbon (wt%) | 0.05 | 0.60 | 0.83 | 1.05 | 1.25 | 1.25 | 1.25 |
| Total | 100.00 | 100.00 | 100.00 | 100.00 | 100.00 | 100.00 | 100.00 |

**Table 2**

| **Dried Film Compositions** | | | | | | | |
|---|---|---|---|---|---|---|---|
| **Ingredient** | **1A** | **2A** | **3A** | **4A** | **5A** | **6A** | **7A** |
| Starch (wt%) | 19.07 | 19.88 | 19.88 | 19.42 | 19.23 | 20.65 | 21.34 |
| Titanium Dioxide (wt%) | 4.77 | 0.00 | 0.00 | 0.00 | 0.00 | 0.00 | 2.59 |
| Hydroxyalkyl Cellulose Polymer(s) (wt%) | 61.96 | 64.60 | 64.60 | 63.13 | 62.50 | 67.10 | 69.35 |
| Propylene Glycol (wt%) | 9.53 | 9.94 | 9.94 | 9.71 | 9.61 | 10.32 | 10.67 |
| Surfactant(s) (wt%) | 2.38 | 2.48 | 2.48 | 2.43 | 2.40 | 2.58 | 2.67 |
| CI Pigment Black 7 (wt%) | 2.04 | -- | -- | -- | -- | -- | -- |
| Charcoal Activated Carbon (wt%) | 0.25 | 3.10 | 4.29 | 5.31 | 6.25 | 6.25 | 6.46 |
| Total | 100.00 | 100.00 | 100.00 | 100.00 | 100.00 | 100.00 | 100.00 |

Each of the films was then shaped into flakes and combined with a substantially clear dentifrice to determine the efficacy of the charcoal flakes suspended in the dentifrice. It was observed that the flakes of the films containing the charcoal were successfully suspended within the substantially clear dentifrice, and no observable bleeding of the charcoal into the ingredients or components of the dentifrice were observed. It was further observed that films containing titanium dioxide were not as dark as films not containing the titanium dioxide.

The present disclosure has been described with reference to exemplary embodiments. Although a limited number of embodiments have been shown and described, it will be appreciated by those skilled in the art that changes may be made in these embodiments within the scope of the claims.

## Claims

1. An oral care composition, comprising:
a dentifrice; and
a film suspended in the dentifrice, wherein the film comprises charcoal, carboxymethyl cellulose and starch;
wherein the charcoal is suspended or entrained within the film; and
wherein the charcoal does not bleed into the dentifrice components until the film is ruptured upon use.

2. The oral care composition of any one of claims 1 , wherein the charcoal is present in an amount of from about 1 wt% to about 10 wt%, based on a total weight of the film.

3. The oral care composition of any one of claims 1 to 2, wherein the charcoal is present in an amount of from about 2 wt% to about 6 wt%, based on a total weight of the film.

4. The oral care composition of any one of claims 1 to 3, wherein the charcoal has a particle size less than or equal to 75 µm.

5. The oral care composition of any one of claims 1 to 4, wherein the charcoal has a particle size of from about 3 µm to about 9 µm.

6. The oral care composition of claim 1, wherein the starch is corn starch.

7. The oral care composition of any one of claims 1 to 6, wherein the film does not include titanium dioxide.

## Patentansprüche

1. Mundpflegezusammensetzung, die Folgendes umfasst:
ein Zahnputzmittel; und
einen in dem Zahnputzmittel suspendierten Film, wobei der Film Holzkohle, Carboxymethylcellulose und Stärke umfasst;
wobei die Holzkohle in dem Film suspendiert oder eingeschlossen ist; und
wobei sich die Holzkohle nicht in die Bestandteile des Zahnputzmittels ausbreitet, bis der Film bei der Verwendung zerrissen wird.

2. Mundpflegezusammensetzung nach einem beliebigen der Ansprüche 1, wobei die Holzkohle in einer Menge von etwa 1 Gew.-% bis etwa 10 Gew.-%, bezogen auf das Gesamtgewicht des Films, vorhanden ist.

3. Mundpflegezusammensetzung nach einem beliebigen der Ansprüche 1 bis 2, wobei die Holzkohle in einer Menge von etwa 2 Gew.-% bis etwa 6 Gew.-%, bezogen auf das Gesamtgewicht des Films, vorhanden ist.

4. Mundpflegezusammensetzung nach einem beliebigen der Ansprüche 1 bis 3, wobei die Holzkohle eine Partikelgröße von weniger als oder gleich 75 µm aufweist.

5. Mundpflegezusammensetzung nach einem beliebigen der Ansprüche 1 bis 4, wobei die Holzkohle eine Partikelgröße von etwa 3 µm bis etwa 9 µm aufweist.

6. Mundpflegezusammensetzung nach Anspruch 1, wobei die Stärke Maisstärke ist.

7. Mundpflegezusammensetzung nach einem beliebigen der Ansprüche 1 bis 6, wobei der Film kein Titandioxid enthält.

## Revendications

1. Composition de soins bucco-dentaires comprenant :
un dentifrice ; et
un film en suspension dans le dentifrice, dans laquelle le film comprend du charbon, de la carboxyméthylcellulose et de l'amidon ;
dans laquelle le charbon est en suspension ou entraîné dans le film ; et
dans laquelle le charbon ne s'infiltre pas dans les constituants du dentifrice jusqu'à ce que le film soit rompu lors de l'utilisation.

2. Composition de soins bucco-dentaires selon l'une quelconque des revendications 1, dans laquelle le charbon est présent en une quantité d'environ 1 % en poids à environ 10 % en poids, par rapport au poids total du film.

3. Composition de soins bucco-dentaires selon l'une quelconque des revendications 1 à 2, dans laquelle le charbon est présent en une quantité d'environ 2 % en poids à environ 6 % en poids, par rapport au poids total du film.

4. Composition de soins bucco-dentaires selon l'une quelconque des revendications 1 à 3, dans laquelle le charbon a une taille de particule inférieure ou égale à 75 µm.

5. Composition de soins bucco-dentaires selon l'une quelconque des revendications 1 à 4, dans laquelle le charbon a une taille de particule d'environ 3 µm à environ 9 µm.

6. Composition de soins bucco-dentaires selon la revendication 1, dans laquelle l'amidon est de l'amidon de maïs.

7. Composition de soins bucco-dentaires selon l'une quelconque des revendications 1 à 6, dans laquelle le film ne comprend pas de dioxyde de titane.
